# NEUE EUROPÄISCHE PATENTSCHRIFT

(11) **EP 2 507 202 B2**
(45) Veröffentlichungstag und Bekanntmachung des Hinweises auf die Entscheidung über den Einspruch: **29.03.2017**
(45) Hinweis auf die Patenterteilung: 08.01.2014
(21) Anmeldenummer: 10785407.7
(22) Anmeldetag: 30.11.2010
(51) Int. Cl.: C07C 209/84, C07C 211/10

(54) **VERFAHREN ZUR DESTILLATION VON GEMISCHEN ENTHALTEND ETHYLENDIAMIN, N-METHYLETHYLENDIAMIN UND WASSER UND DAMIT ERHÄLTLICHE GEMISCHE AUS ETHYLENDIAMIN UND N-METHYLETHYLENDIAMIN MIT EINEM GERINGEN N-METHYLETHYLENDIAMINGEHALT**
METHOD FOR DISTILLING MIXTURES COMPRISING ETHYLENE DIAMINE, N-METHYLETHYLENE DIAMINE, AND WATER, AND MIXTURES OF ETHYLENE DIAMINE AND N-METHYLETHYLENE DIAMINE HAVING A LOW CONTENT OF N-METHYLETHYLENE DIAMINE OBTAINABLE THEREBY
PROCÉDÉ DE DISTILLATION DE MÉLANGES CONTENANT DE L'ÉTHYLÈNE DIAMINE, DE LA N-MÉTHYLÉTHYLÈNE DIAMINE ET DE L'EAU ET MÉLANGES D'ÉTHYLÈNE DIAMINE ET DE N-MÉTHYLÉTHYLÈNE DIAMINE AYANT UNE FAIBLE TENEUR EN N-MÉTHYLÉTHYLÈNE DIAMINE POUVANT ÊTRE OBTENUS SELON CE PROCÉDÉ

(30) Priorität: 02.12.2009 EP 09177776
(43) Veröffentlichungstag der Anmeldung: 10.10.2012
(73) Patentinhaber: BASF SE, 67056 Ludwigshafen am Rhein (DE)
(72) Erfinder: JÖDECKE, Michael, B-2950 Kapellen (BE); PASTRE, Jörg, 64625 Bensheim (DE); HUGO, Randolf, 67246 Dirmstein (DE)
(74) Vertreter: BASF IP Association
(86) Internationale Anmeldenummer: PCT/EP2010/068469
(87) Internationale Veröffentlichungsnummer: WO 2011/067226

(56) Entgegenhaltungen:
- EP-A2- 2 346 810
- WO-A1-2005/014523
- WO-A2-2010/042168
- DE-B- 1 258 413
- US-A- 3 126 383
- US-A- 4 032 411
- US-A- 4 032 411
- KARSTEN ELLER ET AL: "Ullmann's Encyclopedia of industrial Chemistry; 6th ed; A2, Amines aliphatic", 1. Januar 2002 (2002-01-01), ULLMANN'S ENCYCLOPEDIA OF INDUSTRIAL CHEMISTRY, XX, XX, PAGE(S) 1 - 54, XP002525107, in der Anmeldung erwähnt Seite 32, Spalte 1 - Seite 34, Spalte 1
- M. HIRATA ET AL.: 'Vapora-Liquid Equilibria under Elevated Pressures' JOURNAL OF CHEMICAL ENGINEERING OF JAPAN Bd. 2, Nr. 2, 1969, Seiten 143 - 149
- Bulletin de la Société Chimique de France, 1963, 8-9, 1606-1608
- Graphical Darstellung Verhältnis H20:EDA

## Beschreibung

Die vorliegende Erfindung betrifft ein Gemisch aus Ethylendiamin (EDA) und N-Methylethylendiamin (Me-EDA) mit einem geringen Gehalt an Me-EDA. Die vorliegende Erfindung betrifft weiterhin ein Verfahren zur destillativen Aufarbeitung eines Gemisches enthaltend EDA, Me-EDA und Wasser sowie ein Verfahren zur Herstellung eines destillierbaren Gemischs enthaltend EDA, Me-EDA und Wasser, welches sich zur Herstellung von EDA mit einem geringen Me-EDA-Gehalt eignet.

Ethylendiamin (=Diaminoethan) wird vorwiegend als Zwischenprodukt für die Produktion von Bleichaktivatoren, Pflanzenschutzmitteln, Pharmazeutika, Schmiermitteln, Textilharzen, Polyamiden, Papierhilfsmifteln oder Benzinadditiven eingesetzt.

Zur Herstellung von Ethylendiamin (EDA) sind zahlreiche Verfahren bekannt (siehe beispielsweise Ullmann's Encyclopedia of Industrial Chemistry, "Amines, Aliphatic", Absatz 8.1.1, DOI: 10.1002/14356007.a02_001).

Bei der Herstellung von Ethylendiamin kann N-Methylethylendiamin (Me-EDA) durch Nebenreaktionen gebildet werden.

So kann zum Beispiel bei der Umsetzung von Monoethanolamin (MEOA) mit Ammoniak zu EDA durch eine Abbaureaktion von Monoethanolamin direkt Kohlenmonoxid (CO) und Methylamin entstehen (Decarbonylierung). Das Methylamin kann wiederum direkt mit weiterem Monoethanolamin zu Me-EDA reagieren.

Me-EDA kann auch bei der Dimerisierung von Monoethanolamin zu Aminoethylethanolamin (AEEA) entstehen, wenn AEEA direkt durch Decarbonylierung zu Me-EDA abgebaut wird.

Me-EDA kann sich auch bei der Herstellung von Ethylendiamin aus C1-Bausteinen, wie Blausäure und Formaldehyd bilden.

Für die meisten technischen Anwendungen wird vom Markt eine Reinheit für EDA von mindestens 99,5 Gew.-% gefordert. Organische Nebenkomponenten, darunter Me-EDA, dürfen maximal mit einem Anteil von 0,5 Gew.-% enthalten sein. Darüber hinaus darf der Wassergehalt maximal 0,5 Gew.-% betragen.
Insbesondere bei EDA-Herstellungsverfahren, die auf Monoethanolamin oder C1-Bausteinen, wie Blausäure und Formaldehyd basieren, kann ein Gehalt von weniger als 0,5 Gew.-% Me-EDA in der Regel nicht realisiert werden, wenn die Investitions- und Produktionskosten in einem ökonomischen Rahmen verbleiben sollen.
Eine Erhöhung des Anteils an Me-EDA kann auch mit zunehmender Betriebsdauer und Deaktivierung des Katalysators auftreten, der für die EDA-Herstellung eingesetzt wird, da zur Kompensation der geringeren Aktivität des Katalysators in der Regel die Reaktionstemperatur erhöht wird und somit die Bildung von unerwünschten Nebenprodukten zunimmt.

EDA, das herstellungsbedingt einen höheren Me-EDA-Anteil enthält, muss entsprechend aufgearbeitet werden, so dass spezifikationsgemäß ein Anteil von 0.5 Gew.-% an organischen Nebenkomponenten und 0.5 Gew.-% Wasser nicht überschritten wird.

Me-EDA und EDA bilden bei Normaldruck ein engsiedendes azeotropes Gemisch, welches sich im Allgemeinen mit technisch vertretbarem Aufwand nicht trennen lässt.

Bei der Trennung von Wasser und EDA erfolgt in der Regel nur eine geringe Abreicherung von Me-EDA, etwa im Bereich von wenigen 10tel Prozentpunkten.

Da sich geringe Mengen von Me-EDA und Wasser nur sehr schwierig von EDA abtrennen lassen, verbietet sich in der Praxis im Allgemeinen der Einsatz von EDA mit einem höheren Me-EDA-Gehalt.

US 4,032,411 offenbart ein Verfahren zur Entfernung von Wasser aus einer Mischung aus EDA und Wasser und zur Herstellung von EDA mit einem Wassergehalt unterhalb des azeotropen Gleichgewichts durch Destillation der Mischung in Anwesenheit eines Destillationsadjuvants.

Die WO 2005/014523 offenbart die Herstellung von EDA durch Umsetzung von Monoethanolamin (MEOA) mit Ammoniak in Gegenwart eines Katalysators in einem Reaktor und Auftrennung des resultierenden Reaktionsaustrags, wobei bei der Auftrennung erhaltenes Ethylendiamin (EDA) in einem separaten Reaktor in Gegenwart eines Katalysators zu Diethylentriamin (DETA) umgesetzt und der resultierende Reaktionsaustrag der Auftrennung des aus Reaktor resultierenden Reaktionsaustrags zugeführt wird.

Die Aufgabe der vorliegenden Erfindung bestand darin, ein Verfahren zur Aufreinigung von EDA zur Verfügung zu stellen, mit dem EDA aus Verfahren, bei denen herstellungsbedingt ein höherer Anteil an Me-EDA anfällt, zu spezifikationsgerechter Ware verarbeitet werden kann. Dieses Verfahren sollte mit geringen Investitionskosten realisiert und mit geringen Produktionskosten betrieben werden können.
Eine weitere Aufgabe der vorliegenden Erfindung bestand darin, ein Gemisch aus EDA und Me-EDA mit einem geringen Anteil an Me-EDA zur Verfügung zu stellen.

Diese technische Aufgabe wurde durch ein Verfahren zur Destillation eines Gemisches enthaltend Wasser, Ethylendiamin und Methyl-Ethylendiamin gelöst, wobei man das Gemisch in eine Destillationskolonne einleitet, die bei einem Kolonnenkopfdruck von 10 mbar bis 4 bar betrieben wird, dadurch gekennzeichnet dass das Gewichtsverhältnis von Wasser zu Ethylendiamin in dem eingesetzten Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0 ist.

In das erfindungsgemäße Verfahren werden Gemische enthaltend Ethylendiamin, N-Methylethylendiamin und Wasser eingesetzt.

Erfindungsgemäß beträgt das Gewichtsverhältnis von Wasser zu EDA in dem Gemisch, das in das erfindungsgemäße Verfahren eingesetzt wird, a*X : Y, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, vorzugsweise 1,0 bis 2.0 und besonders bevorzugt 1,02 bis 2.0 ist.

Im Folgenden werden Mischungen enthaltend EDA, Wasser und Me-EDA, bei denen das Gewichtsverhältnis von Wasser zu EDA a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, vorzugsweise 1,0 bis 2.0 und besonders bevorzugt 1,02 bis 2.0 ist, als "destillierbare Gemische" bezeichnet.

Wenn das Gewichtsverhältnis von Wasser zu EDA im zu destillierenden Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von weniger als 0,9 ist, so muss dem zu destillierenden Gemisch zusätzlich Wasser zugefügt werden bevor es in das erfindungsgemäße Verfahren eingesetzt wird, so dass das Gewichtsverhältnis von Wasser zu EDA im Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, vorzugsweise 1,0 bis 2.0 und besonders bevorzugt 1,02 bis 2.0 ist.

Als "zu destillierende Gemische" werden demgemäß Mischungen enthaltend EDA, Wasser und Me-EDA bezeichnet, bei denen Gewichtsverhältnis von Wasser zu EDA a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von weniger als 0,9 ist.

Zu destillierende Gemische können erfindungsgemäß durch Zugabe von Wasser in ein destillierbares Gemisch umgewandelt werden.

Demgemäß betrifft die vorliegende Erfindung auch ein Verfahren zur Herstellung eines destillierbaren Gemisches enthaltend EDA und Me-EDA, dadurch gekennzeichnet, dass man zu einem zu destillierenden Gemisch enthaltend EDA und Me-EDA so viel Wasser zufügt, dass nach der Zugabe von Wasser das Gewichtsverhältnis von EDA zu Wasser a*X : Y beträgt, wobei X der Gewichtsanteil von Wasser und Y der Gewichtsanteil von Ethylendiamin am azeotropen Punkt eines Binärgemisches von Wasser und EDA bei dem Kolonnenkopfdruck sind, bei dem die nachfolgende, erfindungsgemäße Destillation des Zwischenprodukts erfolgen kann, und a eine reelle Zahl mit einem Wert von 0,9 bis 2.0, vor- zugsweise 1,0 bis 2.0 und besonders bevorzugt 1,02 bis 2.0 ist.

Der Wassergehalt eines Gemisches enthaltend EDA, Me-EDA und Wasser kann mittels der üblichen Methoden zur Wasserbestimmung, beispielsweise durch Karl-Fischer Titration, bestimmt werden.

Die Zuführung von Wasser erfolgt üblicherweise mittels einer Dosierpumpe, vorzugsweise kontinuierlich.

Die Menge an Wasser, die zur Herstellung eines destillierbaren Gemisches erforderlich ist wird so gewählt, dass nach der Zugabe von Wasser das Gewichtverhältnis von EDA zu Wasser a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, vorzugsweise 1,0 bis 2.0 und mehr und besonders bevorzugt 1,02 bis 2.0 ist.

Die maximal zugefügte Menge an Wasser wird in der Regel so gewählt, dass die Abtrennung des zusätzlich zugegebenen Wassers in einem wirtschaftlichen Rahmen liegt, da die Abtrennung von Wasser den apparativen als auch den energetischen Aufwand bei der Destillation erhöhen kann.
Bevorzugt beträgt nach der Zugabe von Wasser das Gewichtverhältnis von EDA zu Wasser a*X : Y, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind, bei dem die nachfolgende Destillation des destillierbaren Gemischs erfolgt, und a eine reelle Zahl mit einem Wert von 0,9 bis 2,0, bevorzugt 1,0 bis 1,5, besonders bevorzugt 1,01 bis 1,2, und insbesondere bevorzugt 1,02 bis 1,1 ist.

Die Bestimmung des Gewichtsanteils von Wasser am azeotropen Punkt eines Binärgemisches von Wasser und EDA in Abhängigkeit des Drucks ist dem Fachmann geläufig.

So können azeotrope Punkte in Abhängigkeit des Drucks experimentell gemessen werden, wobei die Methoden dem Fachmann bekannt sind. Azeotrope Punkte für das binäre System EDA und Wasser können beispielsweise den in dem Buch "Azeotropic Data Part I" (J. Gmehling, J. Menke, J. Krafczyk, K. Fischer, Wiley-VCH, 2004, Seite 425) aufgeführten Referenzen entnommen werden.

Zudem ist die Lage des binären Gemisches von EDA und Wasser - also eine Zusammenfassung der experimentell bestimmten Werte - ebenfalls in der einschlägigen Literatur beschrieben (siehe "Azeotropic Data Part I" von J. Gmehling, J. Menke, J. Krafczyk, K. Fischer, Wiley-VCH, 2004, Seite 425 oder "Dortmund Data Bank" (http://www.ddbst.de/new/Default.htm).

Der Gewichtsanteil von Wasser am azeotropen Punkt kann weiterhin mit Aktivitätskoeffizienten-Modellen, wie NRTL, in guter Näherung berechnet werden. Diese Methode ist standardmäßig in kommerziellen Simulationsprogrammen, wie Aspen Plus® der Fa. Aspentech implementiert. In den oben erwähnten Quellen für Gemischdaten sind auch Berechnungsparameter, z.B. NRTL-Parameter angegeben, mit deren Hilfe der azeotrope Punkt auch für von den Angaben abweichende Drücke zumindest in guter Näherung berechnet werden kann.
Bevorzugt erfolgt die Berechung des Gewichtsanteils von Wasser am azeotropen Punkt mittels des NRTL-Modells von Aspen. Die Gasphase wird dabei in der Regel ideal gerechnet. Falls mit realer Gasphase gerechnet wird, ist das in den oben genannten Quellen für die Gemischdaten erwähnt. Die Dampfdruckkurven für EDA und Wasser können der Dortmunder Datenbank, anderen Quellen oder anderer Literatur entnommen werden. Damit lässt sich der azeotrope Punkt von EDA und Wasser in Abhängigkeit des Druckes berechnen.
Der Gewichtsanteil von Wasser am azeotropen Punkt, welcher auf Basis von Modellen, wie NRTL berechnet wird, kann innerhalb einer gewissen Fehlergrenze von der experimentell bestimmten azeotropen Zusammensetzung abweichen.
Maßgeblich für die Berechnung der zuzugebenden Menge an Wasser ist jedoch der tatsächliche, in der Realität vorkommende Gewichtsanteil von Wasser am azeotropen Punkt.

Wenn das Gewichtsverhältnis von Wasser zu EDA im destillierbaren Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl im Bereich von 0,9 bis 1,0 ist, so wird in einer bevorzugten Ausführungsform dem destillierbaren Gemisch zusätzlich Wasser zugefügt, so dass ein bevorzugtes destillierbares Gemisch erhalten wird, in dem der Gewichtsanteil von Wasser zu EDA im Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 1,0 bis 2.0 und bevorzugt 1,02 bis 2.0 und ist.
In dieser bevorzugten Ausführungsform wird die maximal zugefügte Menge an Wasser, wie zuvor beschrieben, ebenfalls in der Regel so gewählt, dass die Abtrennung des zusätzlich zugegebenen Wassers in einem wirtschaftlichen Rahmen liegt, da die Abtrennung von Wasser den apparativen als auch den energetischen Aufwand bei der Destillation erhöhen kann.
Bevorzugt beträgt in dieser bevorzugten Ausführungsform nach der Zugabe von Wasser das Gewichtverhältnis von Wasser zu EDA a*X : Y, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind, bei dem die nachfolgende Destillation des destillierbaren Gemischs erfolgt, und a eine reelle Zahl mit einem Wert von 1,0 bis 1,5, bevorzugt 1,01 bis 1,2, und besonders bevorzugt 1,02 bis 1,1 ist.

Bei den Gemischen, die in das erfindungsgemäße Destillationsverfahren oder in das erfindungsgemäße Verfahren zur Herstellung von destillierbaren Gemischen eingesetzt werden, beträgt das Gewichtsverhältnis von EDA zu Me-EDA vorzugsweise 1 : 0,003 bis 1 : 0,2, besonders bevorzugt 1 : 0,005 bis 1 : 0,1 und ganz besonders bevorzugt 1 : 0,01 bis 1 : 0,05.

Gemische, die in das erfindungsgemäße Destillationsverfahren oder in das erfindungsgemäße Verfahren zur Herstellung eines destillierbaren Gemischs eingesetzt werden, können zusätzliche Komponenten, wie höher siedende Amine, nicht umgesetzte Ausgangsstoffe und organische Nebenprodukte enthalten. Unter höher siedenden Amine werden im folgenden Amine bezeichnet, die bei gleichem Druck einen höheren Siedepunkt als EDA aufweisen, beispielsweise Piperazin (PIP), Monoethanolamin, Diethylentriamin, Aminoethylethanolamin, Triethylentetramin (TETA) und höhere Ethylenamine (d.h. Ethylenamine mit einem höheren Siedepunkt als TETA).

Das Gewichtsverhältnis der oben genannten Komponenten in diesen Gemischen beträgt vorzugsweise:
EDA : Wasser = 1 : 0,05 bis 0,8;
EDA : Me-EDA = 1 : 0,003 bis 0,2;
EDA : Ammoniak = 1 : 0 bis 0,05;
EDA : höher siedende Amine = 1 : 0 bis 1,25; und
EDA : organische Nebenprodukte = 1 : 0 bis 0,05;
und besonders bevorzugt:
EDA: Wasser = 1 : 0,1 bis 0,5;
EDA : Me-EDA = 1 : 0,005 bis 0,05;
EDA : Ammoniak = 1 : 0 bis 0,025;
EDA : höher siedende Amine = 1 : 0,05 bis 1; und
EDA : organische Nebenprodukte = 1 : 0,0001 bis 0,025.

Gemische enthaltend Ethylendiamin, N-Methylethylendiamin und Wasser, die in das erfindungsgemäße Destillationsverfahren oder in das erfindungsgemäße Verfahren zur Herstellung eines destillierbaren Gemischs eingesetzt werden können, enthalten bevorzugt weniger als 5 Gew.-% Ammoniak, besonders bevorzugt weniger als 2 Gew.-% Ammoniak, besonders bevorzugt weniger als 1 Gew.-% Ammoniak und insbesondere bevorzugt weniger als 0,5 Gew.-% Ammoniak.

Gemische, die in das erfindungsgemäße Destillationsverfahren oder in das erfindungsgemäße Verfahren zur Herstellung eines destillierbaren Gemisches eingesetzt werden können, werden bevorzugt durch Abtrennung von Wasserstoff und/oder Ammoniak aus einem Reaktionsaustrag erhalten, der bei der Herstellung von EDA anfällt.

Ein solcher Reaktionsaustrag kann beispielsweise durch Umsetzung von MEOA mit Ammoniak oder Umsetzung von Ethylenoxid mit Ammoniak oder Umsetzung von Formaldehyd, Blausäure, Ammoniak und Wasserstoff erhalten werden.
Die Umsetzung von MEOA und Ammoniak ist beispielsweise in der US 2,861,995, der DE-A-1 172 268 und der US 3,112,318 beschrieben. Eine Übersicht über die verschiedenen Verfahrensvarianten der Umsetzung von MEOA mit Ammoniak kann beispielsweise dem PERP Report No. 138 "Alkyl-Amines", SRI International, 03/1981 (insbesondere Seiten 81-99, 117) entnommen werden.
Die Umsetzung von Monoethanolamin mit Ammoniak wird bevorzugt in einem Festbettreaktor an einem Übergangsmetallkatalysator bei 150-250 bar und 160-210°C oder an einem Zeolithkatalysator bei 1-20 bar und 280-380°C durchgeführt.
Bevorzugt verwendete Übergangsmetallkatalysatoren enthalten Ni, Co, Cu, Ru, Re, Rh, Pd oder Pt oder eine Mischung zweier oder mehrerer dieser Metalle auf einem oxidischen Träger (z.B. Al₂O₃, TiO₂, ZrO₂, SiO₂).
Bevorzugte Zeolithkatalysatoren sind Mordenite, Faujasite und Chabazite.
Zur Erzielung einer möglichst hohen EDA-Selektivität wird bei der Übergangsmetallkatalyse im Allgemeinen mit einem molaren Verhältnis von Ammoniak zu Monoethanolamin von 6-20, bevorzugt 8-15, und bei Zeolithkatalyse von im allgemeinen 20-80, bevorzugt 30-50, gearbeitet.
Der MEOA-Umsatz wird in der Regel im Bereich zwischen 10 % und 80 %, bevorzugt 40-60 %, gehalten.
Im kontinuierlichen Betrieb wird bevorzugt eine Katalysatorbelastung im Bereich von 0,3-0,6 kg/(kg*h) (kg MEOA pro kg Kat. pro Stunde) eingestellt.
Zur Aufrechterhaltung der Katalysatoraktivität werden bei Einsatz von Metallkatalysatoren bevorzugt zusätzlich 0,05-0,5 Gew.-% (bezogen auf den Reaktionseintrag MEOA +NH₃+H₂) Wasserstoff in den Reaktor gefahren.

Ein weiterer Weg zur Herstellung eines Reaktionsaustrages ist die direkte Umsetzung von Ethylenoxid mit Ammoniak, welche z.B. in Eur. Chem. News 20 (1971) No. 495, 16 sowie dem oben genannten PERP-Report beschrieben wird.

Die Herstellung eines Reaktionsaustrags kann auch durch die Umsetzung von Formaldehyd, Blausäure, Ammoniak und Wasserstoff erfolgen.
So wird in US-A 2 519 803 ein Verfahren zur Herstellung von Ethylendiamin durch die Hydrierung eines teilweise aufgereinigten wässrigen Reaktionsgemisches beschrieben, welches aus einer Aminierung von Formaldehydcyanhydrin (FACH) resultiert und als Zwischenprodukt Aminoacetonitril enthält. Formaldehydcyanhydrin kann wiederum durch Umsetzung von Formaldehyd mit Blausäure erhalten werden. Eine Verfahrensbeschreibung zur Herstellung von FACH findet sich beispielsweise in der Anmeldung PCT/EP2008/052337, Seite 26 und in der Anmeldung WO-A1-2008/104582, Seite 30 (Variante a) und b)), auf die an dieser Stelle ausdrücklich Bezug genommen wird. DE-A 1 154 121 betrifft ein weiteres Verfahren zur Herstellung von Ethylendiamin, wobei die Edukte Blausäure, Formaldehyd, Ammoniak und Wasserstoff in Gegenwart eines Katalysators in einem so genannten Eintopf-Verfahren zur Reaktion gebracht werden.
Die WO-A1- 2008/104592 betrifft ein Verfahren zur Herstellung von EDA durch Hydrierung von Aminoacetonitril. Aminoacetonitril wird üblicherweise durch Umsetzung von Formaldehydcyanhydrin mit Ammoniak erhalten werden, wobei Formaldehydcyanhydrin wiederum in der Regel aus Blausäure und Ammoniak hergestellt wird. Bevorzugt wird ein Reaktionsaustrag enthaltend EDA und Me-EDA gemäß dem in der WO-A- 2008/104592 beschriebenen Verfahren hergestellt, auf das hiermit ausdrücklich Bezug genommen wird.

Die Reaktionsausträge, die durch die voranstehend beschriebenen Umsetzungen hergestellt werden, enthalten im Allgemeinen Wasserstoff, der in der Regel abgetrennt wird, in dem der Reaktionsaustrag beispielsweise auf 20 bis 30 bar entspannt wird.

Nach der Abtrennung von Wasserstoff enthalten die Reaktionsausträge, die bei der Herstellung von EDA erhalten werden, in der Regel EDA, Wasser, Me-EDA, Ammoniak und im Allgemeinen höher siedende Amine sowie nicht umgesetzte Einsatzstoffe und organische Nebenprodukte.

In einer bevorzugten Ausführungsform weisen die Reaktionsausträge einen relativ hohen Gehalt an Me-EDA auf.
Solche Reaktionsausträge werden bevorzugt erhalten, wenn EDA aus C1-Bausteinen, wie Formaldehyd und Blausäure, hergestellt wird oder wenn der Katalysator, der zur Herstellung von EDA verwendet wird, beispielsweise nach zunehmender Betriebsdauer an Aktivität verliert und dieser Verlust an Aktivität durch Erhöhung der Reaktionstemperatur zu Lasten der EDA-Selektivität kompensiert wird.

In der bevorzugten Ausführungsform, in der die Reaktionsauträge aus der EDA-Herstellung einen relativ hohen Gehalt an Me-EDA aufweisen, beträgt das Gewichtsverhältnis von EDA zu Me-EDA im Reaktionsaustrag, die bei der Herstellung von EDA erhalten werden, vorzugsweise 1 : 0,003 bis 1 : 0,2, besonders bevorzugt 1 : 0,005 bis 1 : 0,1 und ganz besonders bevorzugt 1 : 0,01 bis 1 : 0,05.

Das Gewichtsverhältnis der Komponenten im Reaktionsaustrag beträgt vorzugsweise:
EDA : Wasser = 1 : 0,05 bis 0,8;
EDA : Me-EDA = 1 : 0,003 bis 0,2;
EDA : Ammoniak = 1 : 0,002 bis 20;
EDA : höher siedende Amine = 1 : 0 bis 1,25; und
EDA : organische Nebenprodukte = 1 : 0 bis 0,05.

In einer besonders bevorzugten Ausführungsform werden die Reaktionsausträge durch Umsetzung von MEOA und Ammoniak erhalten, wobei das Gewichtsverhältnis der Komponenten im Reaktionsaustrag:
EDA : Wasser = 1 : 0,1 bis 0,5;
EDA : Me-EDA = 1 : 0,005 bis 0,1;
EDA : Ammoniak = 1 : 3 bis 15;
EDA : höher siedende Amine = 1 : 0,01 bis 1 : 1,0; und
EDA : organische Nebenprodukte = 1 : 0,0001 bis 0,025
und ganz besonders bevorzugt:
EDA : Wasser = 1 : 0,1 bis 0,5;
EDA : Me-EDA = 1 : 0,01 bis 0,05;
EDA : Ammoniak = 1 : 3 bis 15;
EDA : höher siedende Amine = 1 : 0,05 bis 1 : 0,5; und
EDA : organische Nebenprodukte = 1 : 0,0001 bis 0,01
beträgt.

In einer weiteren besonders bevorzugten Ausführungsform werden die Reaktionsausträge durch Umsetzung von Formaldehyd, Blausäure, Ammoniak und Wasserstoff erhalten, wobei das Gewichtsverhältnis der Komponenten im Reaktionsaustrag:
EDA : Wasser = 1 : 0,1 bis 0,5;
EDA : Me-EDA = 1 : 0,005 bis 0,1;
EDA : Ammoniak = 1 : 0,005 bis 0,1;
EDA : höher siedende Amine = 1 : 0,01 bis 1 : 1,0; und
EDA : organische Nebenprodukte = 1 : 0,0001 bis 0,025
und ganz besonders bevorzugt:
EDA : Wasser = 1 : 0,1 bis 0,5;
EDA : Me-EDA = 1 : 0,01 bis 0,05;
EDA : Ammoniak = 1 : 0,01 bis 0,05;
EDA : höher siedende Amine = 1 : 0,05 bis 1 : 0,5; und
EDA : organische Nebenprodukte = 1 : 0,0001 bis 0,01
beträgt.

Neben dem bevorzugten Einsatz von Reaktionsausträgen, die einen relativ hohen Gehalt an Me-EDA aufweisen, können Reaktionsausträge, die EDA, Me-EDA und Wasser enthalten, mit einem geringeren Me-EDA-Gehalt in das erfindungsgemäße Verfahren eingesetzt werden, so dass eine weitere Absenkung des Anteils an Me-EDA erreicht werden kann.

Nach der Abtrennung von Wasserstoff enthalten die Reaktionsausträge aus den verschiedenen EDA-Herstellungsrouten neben EDA, Me-EDA und Wasser üblicherweise zusätzlich Ammoniak.

Die Entfernung des Ammoniaks aus Reaktionsausträgen enthaltend EDA und Me-EDA erfolgt im Allgemeinen in einer Destillationskolonne.
Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.
Die Ammoniakabtrennung erfolgt bevorzugt in einer Druckkolonne, wobei der Kolonnendruck so gewählt wird, dass der Ammoniak mit dem vorhandenen Kühlmedium bei der gegebenen Kühlmediumstemperatur, z.B. Kühlwasser, kondensiert werden kann. Die Ammoniak-Abtrennung erfolgt bevorzugt in einer Destillationskolonne, die Einbauten zur Erhöhung der Trennleistung aufweist.
Die Ammoniak-Abtrennung wird besonders bevorzugt in einer Bodenkolonne durchgeführt, da solche Kolonnen für den Betrieb bei hohem Druck gut geeignet und kostengünstiger als Kolonnen mit geordneten Packungen sind.
Bei einer Bodenkolonne befinden sich im Inneren der Kolonne Zwischenböden, auf denen der Stoffaustausch stattfindet. Beispiele für unterschiedliche Bodentypen sind Siebböden, Tunrielböden, Dualflowböden, Glockenböden oder Ventilböden.
Die destillativen Einbauten können aber auch als geordnete Packung vorliegen, beispielsweise als Blechpackung, wie Mellapak 250 Y oder Montz Pak, Typ B1-250, oder als strukturierte Keramikpackung oder als ungeordnete Packung, z.B. aus Pallringen, IMTP-Ringen (Fa. Koch-Glitsch), Raschig-Superringen, etc. Geordnete oder ungeordnete Packungen können in einem oder, bevorzugt, in mehreren Betten angeordnet sein.

Der Reaktionsaustrag enthaltend EDA und Me-EDA wird vorzugsweise in einem räumlichen Bereich zwischen 30% und 90% der theoretischen Böden der Destillationskolonne zugeführt (von unten gezählt), besonders bevorzugt in einem räumlichen Bereich zwischen 50% und 80% der theoretischen Böden der Destillationskolonne. Beispielsweise kann die Zuführung etwas oberhalb der Mitte der theoretischen Böden erfolgen. Die optimale Zulaufstelle kann vom Fachmann in Abhängigkeit der Ammoniak-Konzentration mit den üblichen Berechnungswerkzeugen ermittelt werden.

Die Anzahl der theoretischen Böden liegt im Allgemeinen im Bereich von 5 bis 30, vorzugsweise 10 bis 20.

Der Kopfdruck beträgt besonders bevorzugt 1 bis 30 bar, besonders bevorzugt 10 bis 20 bar.

Im Kolonnensumpf wird bevorzugt eine Temperatur eingestellt, die oberhalb der Verdampfungstemperatur des Ammoniaks liegt, so dass Ammoniak vollständig oder weitestgehend vollständig in die Gasphase übergeht.
Besonders bevorzugt wird eine Temperatur eingestellt, die nahezu der Siedetemperatur des über Sumpf abzutrennenden Gemisches bei Kolonnensumpfdruck entspricht. Die Temperatur hängt von der Art und Zusammensetzung der im Sumpfprodukt enthaltenen Stoffe ab und kann vom Fachmann mit den üblichen thermodynamischen Berechnungswerkzeugen ermittelt werden. Beispielsweise kann bei einem Kolonnenkopfdruck von 18 bar, bevorzugt eine Kolonnensumpftemperatur von 220 bis 260°C, besonders bevorzugt von 230 bis 250°C eingestellt werden.

Der Kondensator der Destillationskolonne wird in der Regel bei einer Temperatur betrieben, in dem der überwiegende Teil des Ammoniaks bei dem entsprechenden Kopfdruck kondensiert wird. In der Regel liegt die Betriebstemperatur des Kondensators im Bereich von 30 bis 70°C, vorzugsweise 35 bis 50°C.

Der Rücklauf am Kolonnenkopf wird in der Regel so eingestellt, dass die überwiegende Menge der Amine sowie Wasser in der Kolonne zurückgehalten werden, so dass sie praktisch vollständig als Sumpfprodukt erhalten werden. Vorzugsweise wird das am Kondensator anfallende Kondensat zu mehr als 30 %, bevorzugt zu mehr als 49 % in den Kopf der Destillationskolonne zurückgeführt.

Die für die Verdampfung erforderliche Energie wird üblicherweise durch einen Verdampfer im Kolonnensumpf eingetragen.

Im Kondensator fällt als Kondensat überwiegend Ammoniak an.
Das als Kondensat anfallende Ammoniak kann nach einer Aufreinigung oder vorzugsweise direkt als Ausgangsstoff für weitere chemische Synthesen eingesetzt. Beispielsweise kann der als Kondensat anfallende Ammoniak zur Herstellung von Ethylendiamin wiederverwendet werden, in dem der Ammoniak dem Ethylendiamin-Herstellungsprozess zurückgeführt wird.

Als Sumpfaustrag aus der Ammoniak-Abtrennung wird im Allgemeinen ein Gemisch erhalten, welches EDA, Wasser, und Me-EDA und in der Regel höher siedende Amine sowie organische Nebenprodukte enthält.

Wenn das Gewichtsverhältnis von Wasser zu EDA im dem Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von weniger als 0,9 ist, so muss dem zu destillierenden Gemisch, wie voranstehend ausgeführt, zusätzlich Wasser zugefügt werden, bevor es in das erfindungsgemäße Verfahren eingesetzt wird, so dass das Gewichtsverhältnis von Wasser zu EDA im dem in das erfindungsgemäß Verfahren eingesetzte Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, vorzugs- weise 1,0 bis 2.0 und besonders bevorzugt 1,02 bis 2.0 ist.

Wenn das Gewichtsverhältnis von Wasser zu EDA in dem Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, vorzugsweise 1,0 bis 2.0 und besonders bevorzugt 1,02 bis 2.0 ist, so handelt es sich um ein destillierbares Gemisch, das, wie voranstehend beschrieben, direkt in das erfindungsgemäße Destillationsverfahren eingesetzt werden kann.

Wenn das Gewichtsverhältnis von Wasser zu EDA im destillierbaren Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl im Bereich von 0,9 bis 1,0, so wird in einer bevorzugten Ausführungsform dem destillierbaren Gemisch, wie voranstehend beschrieben, zusätzlich Wasser zugefügt, so dass ein bevorzugtes destillierbares Gemisch erhalten wird, in dem der Gewichtsanteil von Wasser zu EDA im Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 1,0 bis 2.0 und bevorzugt 1,02 bis 2.0 ist.

Erfindungsgemäß wird ein destillierbares Gemisch enthaltend EDA, Me-EDA und Wasser, in dem das das Gewichtverhältnis von Wasser zu EDA a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, bevorzugt 1,0 bis 2.0 und besonders bevorzugt 1,02 bis 2.0 ist, in eine Destillationskolonne (Me-EDA-Abtrennung) eingeleitet.

Bevorzugt weist die Destillationskolonne Einbauten zur Erhöhung der Trennleistung auf. Die destillativen Einbauten können vorzugsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252.Y verwendet werden. Vorteilhaft bei der Verwendung dieser destillativen Einbauten sind der geringe Druckverlust und der geringe spezifische Flüssig-Hold-up im Vergleich zu beispielsweise Ventilböden. Die Einbauten können in ein oder mehren Betten vorliegen.

Das destillierbare Gemisch enthaltend EDA, Me-EDA und Wasser wird vorzugsweise in einem räumlichen Bereich zwischen 25% und 75% der theoretischen Böden der Destillationskolonne zugeführt (von unten gezählt), besonders bevorzugt in einem räumlichen Bereich zwischen 30% und 65% der theoretischen Böden der Destillationskolonne. Beispielsweise kann die Zuführung etwas unterhalb der Mitte der theoretischen Böden erfolgen. Die optimale Zulaufstelle kann vom Fachmann mit den üblichen Berechnungswerkzeugen ermittelt werden.

Die Anzahl der theoretischen Böden liegt im Allgemeinen im Bereich von 5 bis 50, vorzugsweise 20 bis 40.

Der Kopfdruck beträgt erfindungsgemäß 10 mbar bis 4 bar, besonders bevorzugt 20 mbar bis 2 bar und ganz besonders bevorzugt 50 mbar bis 200 mbar. In einer weiteren bevorzugten Ausführungsform wird die Kolonne am Kopf bei Atmosphärendruck, ca. 1 bar, betrieben, da bei Atmosphärendruck eine einfache apparative Ausgestaltung der Destillation erfolgen kann.

Im Kolonnensumpf wird bevorzugt eine Temperatur eingestellt, die oberhalb der Verdampfungstemperatur von Wasser liegt, aber unterhalb der Verdampfungstemperatur des azeotropen Binärgemisches aus EDA und Wasser am azeotropen Punkt, so dass die Menge an Wasser in die Gasphase übergeht, die in etwa der Differenz der Wasserkonzentration des zu destillierenden Gemischs (Kolonneneintrag) und dem Gewichtsanteil von Wasser am azeotropen Punkt entspricht.

Beispielsweise kann bei einem Kolonnenkopfdruck von 100 mbar, bevorzugt eine Kolonnensumpftemperatur von 60 bis 90°C, besonders bevorzugt von 65 bis 80°C eingestellt werden.

Der Kondensator der Destillationskolonne wird in der Regel bei einer Temperatur betrieben, in dem der überwiegende Teil des Wassers bei dem entsprechenden Kopfdruck kondensiert wird. In der Regel liegt die Betriebstemperatur des Kondensators im Bereich von 25 bis 70°C, vorzugsweise 30 bis 50°C.

Vorzugsweise wird das am Kondensator anfallende Kondensat zu mehr als 80 %, bevorzugt zu mehr als 90%, in den Kopf der Destillationskolonne zurückgeführt.

Die für die Verdampfung erforderliche Energie wird üblicherweise durch einen Verdampfer im Kolonnensumpf eingetragen.

Im Kondensator fällt als Kondensat welches überwiegend Wasser und Me-EDA enthält.

Im Sumpfaustrag wird in der Regel ein Gemisch erhalten, das EDA und Wasser sowie ggf. höher siedende Amine, wie Piperazin, Monoethanolamin, Diethylentriamin, Aminoethylethanolamin, Triethylentetramin und höhere Ethylenamine, enthält.

Das Verhältnis von Me-EDA zu EDA im Sumpfaustrag ist vorzugsweise geringer als das Verhältnis von Me-EDA zu EDA im zu destillierenden Gemisch, das als Eintrag in die Me-EDA-Abtrennung eingeleitet wird.

Das Gewichtsverhältnis von EDA zu Me-EDA im Sumpfaustrag aus der Me-EDA-Abtrennung beträgt vorzugsweise weniger als 1 : 0,003, besonders bevorzugt weniger als 1 : 0,002 und bevorzugt weniger als 1 : 0,001. Bevorzugt liegt das Gewichtsverhältnis von EDA zu Me-EDA im Sumpfaustrag aus der Me-EDA-Abtrennung im Bereich von 1 : 0 bis 1 : 0,003, besonders bevorzugt 1 : 0,0001 bis 1 : 0,002 und ganz besonders bevorzugt 1 : 0,0002 bis 1 : 0,001.

Überraschenderweise kann die Konzentration von Me-EDA im Sumpfaustrag durch die Destillation eines destillierbaren Gemisches von Me-EDA, EDA und Wasser, bei dem das Gewichtsverhältnis von Wasser zu Ethylendiamin im zu destillierenden Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2,0 ist, stark verringert werden. Dies ist umso überraschender, als Me-EDA und EDA sehr ähnliche Komponenten sind, welche ein engsiedendes azeotropes Gemisch bilden. Die Zugabe von Wasser erhöht offenbar die Flüchtigkeit von Me-EDA im Vergleich zu EDA.

Das Gemisch, das als Sumpfaustrag aus der Me-EDA-Abtrennung erhalten wird, enthält überwiegend Wasser und EDA, sowie ggf. höher siedende Amine, wie Piperazin, Monoethanolamin, Diethylentriamin, Aminoethylethanolamin, Triethylentetramin und höhere Ethylenamine.

Zur Reduzierung des Wassergehalts wird der Sumpfaustrag aus der Me-EDA-Abtrennung im Allgemeinen einer weiteren Destillationsstufe zugeführt ("Wasser-Abtrennung").

Die Entfernung von Wasser aus dem Gemisch enthaltend EDA und Wasser erfolgt im Allgemeinen in einer Destillationskolonne, die so betrieben wird, dass am Kolonnenkopf überwiegen Wasser anfällt und EDA sowie die höher siedenden Amine am Kolonnensumpf entnommen werden können.

Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

Die Wasser-Abtrennung erfolgt in einer Destillationskolonne, die in der Regel Einbauten zur Erhöhung der Trennleistung aufweist.
Die Wasser-Abtrennung wird bevorzugt in einer Bodenkolonne durchgeführt, da solche Kolonnen für den Betrieb bei hohem Druck gut geeignet sind.
Bei einer Bodenkolonne befinden sich im Inneren der Kolonne Zwischenböden, auf denen der Stoffaustausch stattfindet. Beispiele für unterschiedliche Bodentypen sind Siebböden, Tunnelböden, Dualflowböden, Glockenböden oder Ventilböden.
Die destillativen Einbauten können aber auch als geordnete Packung vorliegen, beispielsweise als Blechpackung, wie Mellapak 250 Y oder Montz Pak, Typ B1-250, oder als strukturierte Keramikpackung.

Das destillierbare Gemisch enthaltend EDA und Wasser wird vorzugsweise in einem räumlichen Bereich zwischen 40% und 90%der theoretischen Böden der Destillationskolonne zugeführt (von unten gezählt), besonders bevorzugt in einem räumlichen Bereich zwischen 50% und 75% der theoretischen Böden der Destillationskolonne. Beispielsweise kann die Zuführung etwas oberhalb der Mitte der theoretischen Böden erfolgen. Die optimale Zulaufstelle kann vom Fachmann mit den üblichen Berechnungswerkzeugen ermittelt werden. Die Anzahl der theoretischen Böden liegt im Allgemeinen im Bereich von 10 bis 80, vorzugsweise 30 bis 60.

Der Kopfdruck beträgt besonders bevorzugt 4 bis 30 bar, besonders bevorzugt 6 bis 10 bar. Bei diesen Drücken liegt bildet das binäre Gemisch von Wasser und EDA kein Azeotrop.

Im Kolonnensumpf wird bevorzugt eine Temperatur eingestellt, die oberhalb der Verdampfungstemperatur von Wasser aber unterhalb der Verdampfungstemperatur von EDA liegt. Beispielsweise kann bei einem Kolonnenkopfdruck von 8 bar (abs.), bevorzugt eine Kolonnensumpftemperatur von 180 bis 250°C, besonders bevorzugt von 210 bis 230°C eingestellt werden.

Der Kondensator der Destillationskolonne wird in der Regel bei einer Temperatur betrieben, in dem der überwiegende Teil des Wassers bei dem entsprechenden Kopfdruck kondensiert wird. In der Regel liegt die Betriebstemperatur des Kondensators im Bereich von 150 bis 230°C, vorzugsweise 160 bis 180°C.

Vorzugsweise wird das am Kondensator anfallende Kondensat zu mehr als 50 %, bevorzugt zu mehr als 65 %, in den Kopf der Destillationskolonne zurückgeführt.

Die für die Verdampfung erforderliche Energie wird üblicherweise durch einen Verdampfer im Kolonnensumpf eingetragen.

Im Kondensator fällt ein Kondensat an, welches überwiegend Wasser enthält. Das so erhaltene Wasser kann im Allgemeinen direkt der Entsorgung zugeführt werden, beispielsweise durch Einleiten in eine Abwasseraufbereitungsanlage.

Im Sumpfaustrag der Wasser-Abtrennung wird in der Regel ein Gemisch erhalten, das EDA und ggf. sonstige Amine, wie Piperazin, Monoethanolamin, Diethylentriamin, Aminoethylethanolamin, Triethylentetramin und höhere Ethylenamine, enthält.

Vorzugsweise enthält der Sumpfaustrag aus der Wasser-Abtrennung weniger 0,5 Gew.-% Wasser, vorzugsweise weniger als 0,4 Gew.-% und besonders bevorzugt weniger als 0,3 Gew.-% Wasser.

In der Regel enthält der Sumpfaustrag aus der Wasserabtrennung neben EDA noch Amine, die bei gleichem Druck einen höheren Siedepunkt als EDA aufweisen, sogenannte "höher siedende Amine", wie Piperazin, Monoethanolamin, Diethylentriamin, Aminoethylethanolamin, Triethylentetramin und höhere Ethylenamine.
Die im Sumpfaustrag aus der Wasser-Abtrennung enthaltenen höher siedenden Amine werden im Allgemeinen in ein oder mehreren anschließenden Destillationsstufen abgetrennt.

Der Sumpfaustrag aus der Wasser-Abtrennung kann in einer oder mehreren weiteren Destillationsstufen in die entsprechenden aminhaltigen Fraktionen aufgetrennt werden.

Üblicherweise wird der Sumpfaustrag aus der Wasser-Abtrennung in eine weitere Destillationskolonne eingeleitet (EDA/PIP-Abtrennung), die so betrieben wird, dass EDA und Piperazin am Kolonnenkopf anfallen und die anderen höher siedenden Amine am Kolonnensumpf abgezogen werden können. Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Betten vorliegen.

Der Sumpfaustrag aus der Wasser-Abtrennung wird vorzugsweise in einem räumlichen Bereich zwischen 25% und 75% der theoretischen Böden der Destillationskolonne zugeführt (von unten gezählt), besonders bevorzugt in einem räumlichen Bereich zwischen 30% und 65% der theoretischen Böden der Destillationskolonne. Beispielsweise kann die Zuführung etwas oberhalb der Mitte der theoretischen Böden erfolgen. Die optimale Zulaufstelle kann vom Fachmann mit den üblichen Berechnungswerkzeugen ermittelt werden.

Die Anzahl der theoretischen Böden liegt im Allgemeinen im Bereich von 30 bis 90, vorzugsweise 40 bis 70.

Der Kopfdruck beträgt besonders bevorzugt 0,1 bis 10 bar, besonders bevorzugt 0,5 bis 5 bar. Ganz besonders bevorzugt wird die Kolonne bei Normaldruck betrieben.

Im Kolonnensumpf wird bevorzugt eine Temperatur eingestellt, die oberhalb der Verdampfungstemperatur von EDA aber unterhalb der Verdampfungstemperatur von DE-TA liegt. Beispielsweise kann bei einem Kolonnenkopfdruck von 1 bar (abs.), bevorzugt eine Kolonnensumpftemperatur von 170 bis 220°C, besonders bevorzugt von 180 bis 210°C eingestellt werden.

Der Kondensator der Destillationskolonne wird in der Regel bei einer Temperatur betrieben, in dem der überwiegende Teil des EDA-Piperazin-Gemisches bei dem entsprechenden Kopfdruck kondensiert wird. In der Regel liegt die Betriebstemperatur des Kondensators im Bereich von 50 bis 150°C, vorzugsweise 110 bis 130°C.

Vorzugsweise wird das am Kondensator anfallende Kondensat zu mehr als 30 %, bevorzugt zu mehr als 50 Gew.-%, in den Kopf der Destillationskolonne zurückgeführt.

Die für die Verdampfung erforderliche Energie wird üblicherweise durch einen Verdampfer im Kolonnensumpf eingetragen.

Im Sumpfaustrag wird in der Regel ein Gemisch erhalten, das höher siedende Amine, wie Diethylentriamin, Aminoethylethanolamin, Triethylentetramin und höhere Ethylenamine, enthält. Der Sumpfaustrag kann in weiteren Destillationsstufen in die einzelnen Komponenten oder einzelne Fraktionen aufgetrennt werden.

Im Kondensator fällt ein Kondensat an, welches überwiegend EDA und Piperazin, aber nur sehr geringe Mengen an Me-EDA enthält.

Das Kondensat ist im Allgemeinen überwiegend frei von anderen höher siedenden Aminen. Der Anteil an höher siedenden Aminen (ohne Piperazin) beträgt in der Regel weniger als 0,2 Gew.-%, vorzugsweise weniger als 0,1 Gew.-% und besonders bevorzugt weniger als 0,05 Gew.-%.

Der Kopfaustrag aus der EDA/PIP-Abtrennung wird üblicherweise in eine weitere Destillationskolonne eingeleitet (EDA-Abtrennung), die so betrieben wird, dass EDA am Kolonnenkopf anfällt und Piperazin am Kolonnensumpf abgezogen werden kann. Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Betten vorliegen.

Der Kopfaustrag aus der EDA/PIP-Abtrennung wird vorzugsweise in einem räumlichen Bereich zwischen 25% und 75% der theoretischen Böden der Destillationskolonne zugeführt (von unten gezählt), besonders bevorzugt in einem räumlichen Bereich zwischen 30% und 65% der theoretischen Böden der Destillationskolonne. Beispielsweise kann die Zuführung etwas unterhalb der Mitte der theoretischen Böden erfolgen. Die optimale Zulaufstelle kann vom Fachmann mit den üblichen Berechnungswerkzeugen ermittelt werden.

Die Anzahl der theoretischen Böden liegt im Allgemeinen im Bereich von 10 bis 70, vorzugsweise 20 bis 50.

Der Kopfdruck beträgt besonders bevorzugt 0,1 bis 10 bar, besonders bevorzugt 0,5 bis 5 bar. Ganz besonders bevorzugt wird die Kolonne bei Normaldruck betrieben.

Im Kolonnensumpf wird bevorzugt eine Temperatur eingestellt, die oberhalb der Verdampfungstemperatur von EDA aber unterhalb der Verdampfungstemperatur von Piperazin liegt. Beispielsweise kann bei einem Kolonnenkopfdruck von 1 bar(abs.), bevorzugt eine Kolonnensumpftemperatur von 120 bis 200°C, besonders bevorzugt von 140 bis 160°C eingestellt werden.

Der Kondensator der Destillationskolonne wird in der Regel bei einer Temperatur betrieben, in dem der überwiegende Teil des EDA-Piperazin-Gemisches bei dem entsprechenden Kopfdruck kondensiert wird. In der Regel liegt die Betriebstemperatur des Kondensators im Bereich von 50 bis 150°C, vorzugsweise 110 bis 130°C.

Vorzugsweise wird das am Kondensator anfallende Kondensat zu mehr als 30 %, bevorzugt zu mehr als 50 Gew.-%, in den Kopf der Destillationskolonne zurückgeführt.

Die für die Verdampfung erforderliche Energie wird üblicherweise durch einen Verdampfer im Kolonnensumpf eingetragen.

Im Sumpfaustrag wird in der Regel Piperazin erhalten.

Im Kondensator fällt ein Kondensat an, welches überwiegend EDA, aber nur sehr geringe Mengen an Me-EDA enthält.

Das Kondensat ist im Allgemeinen überwiegend frei von höher siedenden Aminen, inklusive Piperazin. Der Anteil an höher siedenden Aminen, inklusive Piperazin, beträgt in der Regel weniger als 0,5 Gew.-%, vorzugsweise weniger als 0,3 Gew.-% und besonders bevorzugt weniger als 0,2 Gew.-%.

Mittels des erfindungsgemäßen Verfahrens kann ein Gemisch von EDA und Me-EDA erhalten werden, wobei das Gemisch mindestens 99,5 Gew.-% EDA enthält und die Konzentration von Me-EDA weniger als 0,4 Gew.-%, bevorzugt weniger als 0,3 Gew.-%, besonders bevorzugt weniger als 0,2 Gew.-% und insbesondere bevorzugt weniger als 0,1 Gew.-% beträgt.
Mittels des erfindungsgemäßen Verfahrens kann ein Gemisch von EDA und Me-EDA erhalten werden, wobei das Gemisch mindestens 99,5 Gew.-% EDA enthält und die Konzentration von Me-EDA im Bereich von 0 bis 0,4 Gew.-%, vorzugsweise 0,005 bis 0,3 Gew.-%, besonders bevorzugt von 0,01 bis 0,15 Gew.-% beträgt.

Mittels des erfindungsgemäßen Verfahrens können auch Gemische von EDA und Me-EDA erhalten werden, die einen sehr geringen Me-EDA-Anteil aufweisen.
Demgemäß betrifft vorliegende Erfindung auch Gemisch von EDA und Me-EDA, dadurch gekennzeichnet, dass das Gemisch mindestens 99,5 Gew.-% EDA enthält und die Konzentration von Me-EDA im Bereich von 0,005 bis 0,15 Gew.-%, bevorzugt 0,008 bis 0,1 Gew.-% und besonders bevorzugt im Bereich von 0,01 bis 0,05 Gew.-% liegt.

Das erfindungsgemäße Gemisch ist für Anwendungen vorteilhaft geeignet, bei denen es auf eine sehr hohe Reinheit des EDA ankommt.
Das erfindungsgemäße Gemisch kann beispielsweise zur Herstellung von hochmolekularen Polymeren, wie Polyamiden, eingesetzt werden, da die Funktionalität des EDA nicht durch die Bildung von Me-EDA verringert wird. Beispielsweise kann das erfindungsgemäße Gemisch auch als Elektronikchemikalie oder als hochreine Chemikalie zur Anwendung auf dem Gebiet der Pflanzenschutzmittel, Pestizide, Epoxyharze, Komplexbildner oder für Anwendungen in der Lederindustrie, der Papierindustrie oder der Waschmittelindustrie eingesetzt werden. Die Verwendung von hochreinen Chemikalien erhöht die Ausbeute an Endprodukt, verringert die Konzentration von unerwünschten Nebenprodukten und kann weiterhin zu einer Verbesserung der Anwendungs- und Verarbeitungseigenschaften in den betreffenden Anwendungsgebieten führen.

Das erfindungsgemäße Gemisch kann wie voranstehend beschrieben hergestellt werden, in dem man ein Gemisch enthaltend EDA, Me-EDA und Wasser in eine Destillationskolonne zur Abtrennung von Me-EDA einleitet, die bei einem Kolonnenkopfdruck von 10 mbar bis 4 bar betrieben wird, dadurch gekennzeichnet dass das Gewichtsverhältnis von Wasser zu Ethylendiamin im zu destillierenden Gemisch a*X : Y beträgt, wobei X der Gewichtsanteil von Wasser und Y der Gewichtsanteil von Ethylendiamin am azeotropen Punkt eines Binärgemisches von Wasser und EDA bei dem betreffenden Kolonnenkopfdruck sind und a eine reelle Zahl mit einem Wert von 0,9 bis 2.0, bevorzugt 1,0 bis 2.0 und besonders bevorzugt 1,02 bis 2.0 ist, und man den Sumpfaustrag aus der Me-EDA-Abtrennung in eine weitere Destillationskolonne zur Wasser-Abtrennung einleitet, in der am Kopf ein wässriges Destillat und im Sumpf ein wasserabgereichertes Produkt anfällt, und man das wasserabgereicherte Sumpfprodukt aus der Wasser-Abtrennung in eine bzw. mehrere Destillationsstufe(n) zur EDA-Abtrennung einleitet, wobei das erfindungsgemäße Gemisch am Kopf der bzw. einer der Kolonnen erhalten wird.

Mittels des erfindungsgemäßen Verfahrens zur Abtrennung von Me-EDA aus einem Gemisch enthaltend EDA, Me-EDA und Wasser, welches bei der Herstellung von EDA anfällt, kann ein spezifikationsgerechtes EDA mit einem Gehalt von mindestens 99,5 Gew.-% EDA auch dann enthalten werden, wenn sich bei der Herstellung von EDA größere Mengen an Me-EDA bilden. Dies kann beispielsweise dann der Fall sein, wenn EDA aus C1-Bausteinen, wie Formaldehyd und Blausäure hergestellt wird, oder wenn Katalysatoren mit zunehmender Betriebsdauer eine partielle Deaktivierung zeigen und zur Kompensation der Deaktivierung die Reaktionstemperatur erhöht werden muss. Die Erhöhung der Temperatur hat in der Regel zufolge, dass sich die Selektvität in Bezug auf die Herstellung von EDA verschlechtert und vermehrt Me-EDA als Nebenprodukt gebildet wird. Somit ermöglicht das erfindungsgemäße Verfahren auch die Erhöhung der Einsatzzeiten von Katalysatoren bei der Herstellung von EDA.
Mittels des erfindungsgemäßen Verfahrens kann weiterhin ein hochreines EDA erhalten werden, das sich mit verbesserter Ausbeute und weniger Nebenreaktionen als Ausgangsstoff in einer Vielzahl von Anwendungen einsetzen lässt.

Das erfindungsgemäße Verfahren wird anhand von nachfolgenden Beispielen erläutert.

### Beispiel 1:

### Destillation gemäß Stand der Technik

Der Austrag aus dem Reaktor der Ethylenaminsynthese enthält 5,1 Gew.-% Wasser, 11,7 Gew-.% EDA, 300 Gew.-ppm Me-EDA, 1,2 Gew.-% Piperazin, 9,6 Gew.-% Monoethanolamin, 1,6 Gew.-% DETA, 2,3 Gew.-% höher als DETA siedende Ethylenamine bzw. Hochsieder, der Rest ist Ammoniak. Nach Ammoniakabtrennung in einer Druckdestillation bei 18 bar wird in einer weiteren Druckdestillation mit 50 theoretischen Trennstufen bei 8 bar Wasser über Kopf abgetrennt. Der Sumpfaustrag mit den Ethylenaminen enthält ca. 1200 Gew.ppm Wasser und ca. 800 Gew.ppm Me-EDA. Nach weiteren Destillationsschritten unter Normaldruck, bei denen die Amine in die Reinkomponenten aufgetrennt werden, erhält man eine EDA-Fraktion mit mehr als 99,5 Gew.-% EDA, ca. 2500 Gew.ppm Wasser und 1800 Gew.ppm Me-EDA, d.h. die EDA Fraktion ist spezifikationsgerecht.

### Beispiel 2:

### Destillation gemäß Stand der Technik bei höherem Me-EDA-Gehalt

Der Austrag aus dem Reaktor der Ethylenaminsynthese enthält 5,1 Gew.-% Wasser, 11,7 Gew.-% EDA, 1500 Gew.-ppm (bzw. 2000 Gew.-ppm) Me-EDA, 1,2 Gew.-% Piperazin, 9,6 Gew.-% Monoethanolamin, 1,6 Gew.-% DETA, 2,3 Gew.-% höher als DE-TA siedende Ethylenamine bzw. Hochsieder, der Rest ist Ammoniak. Die Zusammensetzung entspricht weitgehend Beispiel 1, jedoch ist der Anteil an Me-EDA auf 1500 Gew.ppm bzw. 2000 Gew. ppm erhöht. Nach Ammoniakabtrennung in einer Druckdestillation bei 18 bar wird in einer weiteren Druckdestillation bei 8 bar Wasser über Kopf abgetrennt. Bei gleicher Anzahl von theoretischen Trennstufen wie in Beispiel 1 gelingt es für beide Me-EDA-Konzentrationen allenfalls unter erheblich höherem Energieaufwand, das Wasser soweit abzutrennen, dass schließlich eine spezifikationsgerechte EDA-Fraktion mit mindestens 99,5 Gew.-% EDA erzeugt werden kann. Gleichzeitig wird das abdestillierte Wasser erheblich höher mit EDA und Me-EDA belastet (ca. 5 Gew.-% statt 2000 ppm). Bei 2000 Gew.ppm Me-EDA im Reaktionsaustrag ist die geforderte Reinheit für EDA nur noch unter erhöhtem Energieaufwand und Inkaufnahme von Produktverlust (verringerte EDA Destillationsausbeute) zu erzielen. Gleichzeitig wird die Aminbelastung des abdestillierten Wasserstroms zunehmend größer und die Entsorgung entsprechend aufwendiger, da der Amingehalt (EDA und Me-EDA) bei einem Me-EDA-Gehalt von 2000 ppm bereits 12 Gew.-% erreicht, drei viertel davon EDA.

### Beispiel 3:

### Erfindungsgemäße Destillation

Der Austrag aus dem Reaktor der Ethylenaminsynthese enthält 5,1 Gew.-% Wasser, 11,7 Gew.-% EDA, 1500 Gew.-ppm Me-EDA, 1,2 Gew.-% Piperazin, 9,6 Gew.-% Monoethanolamin, 1,6 Gew.-% DETA, 2,3 Gew.-% höher als DETA siedende Ethylenamine bzw. Hochsieder, der Rest ist Ammoniak, d.h. die Zusammensetzung entspricht Beispiel 2. Nach Ammoniakabtrennung in einer Druckdestillation bei 18 bar wird zunächst in einer Destillationskolonne bei einem Kopfdruck von 100 mbar (abs) eine Mischung von Wasser (85 Gew.-%), Me-EDA (14 Gew.-%) und EDA (1,0 Gew.-%) über Kopf abgetrennt. Bei 0,1 bar (abs.) liegt das binäre Azeotrop EDA-Wasser bei ca. 27,3 Gew.-% Wasser. Das Verhältnis von Wasser zu EDA im Zulaufstrom von 5,1 % : 11,7%, d.h, 30,4% Wasser bezogen auf das Binärsystem, liegt höher, als im binären Azeotrop. Daher ist es nicht erforderlich weiteres Wasser zur Destillation zuzufahren. In einer anschließenden Druckdestillation mit 50 theoretischen Trennstufen wird bei 8 bar weiteres Wasser über Kopf abgetrennt, analog zu Beispiel 1. Der Sumpfaustrag mit den Ethylenaminen enthält ca. 1500 Gew.ppm Wasser und ca. 40 Gew.ppm Me-EDA. Nach weiteren Destillationsschritten, bei denen die Amine in die Reinkomponenten aufgetrennt werden, erhält man eine EDA-Fraktion mit ca. 99,65 Gew.-% EDA, ca. 2900 Gew.ppm Wasser und 90 Gew.ppm Me-EDA, d.h. die EDA Fraktion ist spezifikationsgerecht. Bei gleichem Me-EDA-Anteil im Reaktoraustrag wie in Beispiel 2 wird eine höhere Reinheit für EDA erreicht, wobei die insgesamt aufzuwendende Energie niedriger ist als in Beispiel 2. Die Wasserabtrennung kann gegenüber den Beispielen 1 oder 2 weniger scharf gefahren werden. Über den Abwasserstrom geht kaum EDA verloren

### Beispiel 4:

### Erfindungsgemäße Destillation eines Me-EDA enthaltenden Gemisches von Ethylenaminen

Ein Gemisch enthält 18,2 Gew.-% Wasser, 72,7 Gew.-% EDA, 9,1 Gew.-% Me-EDA. Bei 0,1 bar (abs.) liegt das binäre Azeotrop EDA-Wasser bei ca. 27,3 Gew.-% Wasser. Das Verhältnis von Wasser zu EDA im azeotropen Punkt beträgt also 27,3/(100-27,3)=0,375. Das Verhältnis von Wasser zu EDA im Zulaufstrom von 18,2 % : 72,7 % = 0,250 (20,0% Wasser bezogen auf das Binärsystem) liegt niedriger, als im binären Azeotrop. Eine erfindungsgemäße Trennung ist so nicht möglich. Dem Zulauf wird deshalb weiteres Wasser zugemischt, und zwar 13 Gew.-Anteile bezogen auf den oben genannten Zulaufstrom, d.h. auf einen Zulaufstrom von 1000 kg/h (darin enthaltend 182 kg/h Wasser und 727 kg/h EDA) werden weitere 130 kg/h Wasser zugemischt. Insgesamt sind dann also 312 kg/h Wasser im Zulauf enthalten. Das Verhältnis von Wasser zu EDA beträgt erfindungsgemäß 312 : 727 = 0,429 (30,0% bezogen auf das Binärsystem). Das Verhältnis von Wasser zu EDA im Zulaufstrom entspricht dann a = 1,14 mal dem Verhältnis von Wasser zu EDA im azeotropen Punkt. Der Kopfproduktstrom der Kolonne enthält ca. 35 Gew.-% Wasser, 1 Gew.-% EDA und 64 Gew.-% Me-EDA. Der Sumpfproduktstrom enthält ca. 26,6 Gew.-% Wasser, 73,3 Gew.-% EDA und 0,1 Gew.-% Me-EDA. In einer anschließenden Druckdestillation mit 30 theoretischen Trennstufen wird bei 14 bar (abs.) weiteres Wasser über Kopf abgetrennt. Der Sumpfaustrag enthält ca. 99,66 Gew.-% EDA, 2500 Gew.ppm Wasser und ca. 900 Gew.ppm Me-EDA.

### Beispiel 5:

### Erfindungsgemäße Destillation eines Me-EDA enthaltenden Gemisches von Ethylenaminen

Ein Gemisch enthält 18,2 Gew.-% Wasser, 72,7 Gew.-% EDA, 9,1 Gew.-% Me-EDA. Es soll bei Normaldruck so getrennt werden, dass die EDA-Fraktion mindestens 99,5 Gew.-% EDA enthält. Bei 1 bar (abs.) liegt das binäre Azeotrop EDA-Wasser bei ca. 14,7 Gew.-% Wasser.. Das Verhältnis von Wasser zu EDA im azeotropen Punkt beträgt 14,7/(100-14,7) = 0,172. Das Verhältnis von Wasser zu EDA im Zulaufstrom von 18,2 % : 72,7 % = 0,250 (20,0 % Wasser bezogen auf das Binärsystem) liegt höher, als im binären Azeotrop. Das Verhältnis von Wasser zu EDA im Zulaufstrom entspricht dann a = 1,45 * dem Verhältnis von Wasser zu EDA im azeotropen Punkt. Dem Zulauf muß somit kein weiteres Wasser zugemischt werden. Der Kopfproduktstrom der Kolonne enthält ca. 38,9 Gew.-% Wasser, 1 Gew.-% EDA und 60,1 Gew.-% Me-EDA. Der Sumpfproduktstrom enthält ca. 14,5 Gew.-% Wasser, 85,4 Gew.-% EDA und 0,1 Gew.-% Me-EDA. In einer anschließenden Druckdestillation mit 30 theoretischen Trennstufen wird bei 14 bar (abs.) weiteres Wasser über Kopf abgetrennt. Der Sumpfaustrag enthält ca. 99,65 Gew.-% EDA, 2500 Gew.ppm Wasser und ca. 1000 Gew.ppm Me-EDA. Statt über Sumpf kann die EDA Fraktion in praktisch gleicher Reinheit über einen dampfförmigen Seitenabzug im Abtriebsteil der Kolonne gewonnen werden.

## Patentansprüche

1. Verfahren zur Destillation eines Gemisches enthaltend Wasser, Ethylendiamin und N-Methylethylendiamin, wobei man das Gemisch in eine Destillationskolonne einleitet, die bei einem Kolonnenkopfdruck von 10 mbar bis 4 bar betrieben wird, **dadurch gekennzeichnet dass** das Gewichtsverhältnis von Wasser zu Ethylendiamin im dem eingesetzten Gemisch a*X : Y beträgt, wobei X der Gewichtsanteil von Wasser und Y der Gewichtsanteil von Ethylendiamin am azeotropen Punkt eines Binärgemisches von Wasser und Ethylendiamin bei dem betreffenden Kolonnenkopfdruck sind und a eine reelle Zahl mit einem Wert von 0,9 bis 2,0 ist.

2. Verfahren nach Anspruch 1, **dadurch gekennzeichnet, dass** a eine reelle Zahl mit einem Wert von 1,01 bis 1,2 ist.

3. Verfahren nach mindestens einem der Ansprüche 1 bis 2, **dadurch gekennzeichnet, dass** das in das Verfahren eingesetzte Gemisch weniger als 5 Gew.-% Ammoniak enthält.

4. Verfahren nach mindestens einem der Ansprüche 1 bis 3, **dadurch gekennzeichnet, dass** das Gewichtsverhältnis von Ethylendiamin zu N-Methylethylendiamin in dem eingesetzten Gemisch im Bereich von 1 : 0,005 bis 1 : 0,1 beträgt.

5. Verfahren nach mindestens einem der Ansprüche 1 bis 4, **dadurch gekennzeichnet, dass** der Kolonnenkopfdruck 50 mbar bis 200 mbar beträgt.

6. Verfahren nach mindestens einem der Ansprüche 1 bis 4, **dadurch gekennzeichnet, dass** der Kolonnenkopfdruck Atmosphärendruck beträgt.

7. Verfahren nach mindestens einem der Ansprüche 1 bis 6, **dadurch gekennzeichnet, dass** das in das Verfahren eingesetzte Gemisch noch weitere Amine enthält, die einen höheren Siedepunkt als Ethylendiamin aufweisen, wobei das Gewichtsverhältnis von Ethylendiamin zu den höher siedenden Aminen im Bereich von 1 : 0,1 bis 1 : 1 beträgt.

8. Verfahren nach mindestens einem der Ansprüche 1 bis 7, **dadurch gekennzeichnet, dass** das in das Verfahren eingesetzte Gemisch aus der Umsetzung von Ethylendiamin aus Ethanolamin und Ammoniak erhalten wurde.

9. Verfahren nach mindestens einem der Ansprüche 1 bis 8, **dadurch gekennzeichnet, dass** das in das Verfahren eingesetzte Gemisch aus der Umsetzung von Formaldehyd, Blausäure, Ammoniak und Wasserstoff erhalten wurde.

10. Verfahren zur Herstellung eines destillierbaren Gemischs enthaltend Ethylendiamin und N-Methylethylendiamin, **dadurch gekennzeichnet, dass** man zur einer Mischung enthaltend Ethylendiamin und N-Methylethylendiamin so viel Wasser zufügt, dass nach der Zugabe von Wasser das Gewichtsverhältnis von Ethylendiamin zu Wasser a*X : Y beträgt, wobei X der Gewichtsanteil von Wasser und Y der Gewichtsanteil von Ethylendiamin am azeotropen Punkt eines Binärgemisches von Wasser und Ethylendiamin bei dem Kolonnenkopfdruck sind, bei dem eine nachfolgende Destillation des destillierbaren Gemischs gemäß einem der Ansprüche 1 bis 9 erfolgen kann, und a eine reelle Zahl mit einem Wert von 0,9 bis 2,0 ist.

11. Verfahren nach Anspruch 10, **dadurch gekennzeichnet, dass** das destillierbare Gemisch in einer Destillationskolonne bei einem Druck von 10 mbar bis 4 bar destilliert wird.

## Claims

1. A process for distilling a mixture comprising water, ethylenediamine and N-methylethylenediamine, by introducing the mixture into a distillation column which is operated at a column top pressure of 10 mbar to 4 bar, wherein the weight ratio of water to ethylenediamine in the mixture used is a*X : Y where X is the proportion by weight of water and Y is the proportion by weight of ethylenediamine at the azeotropic point of a binary mixture of water and ethylenediamine at the column top pressure in question, and a is a real number with a value of 0.9 to 2.0.

2. The process according to claim 1, wherein a is a real number with a value of 1.01 to 1.2.

3. The process according to at least one of claims 1 and 2, wherein the mixture used in the process comprises less than 5% by weight of ammonia.

4. The process according to at least one of claims 1 to 3, wherein the weight ratio of ethylenediamine to N-methylethylenediamine in the mixture used is in the range from 1:0.005 to 1:0.1.

5. The process according to at least one of claims 1 to 4, wherein the column top pressure is 50 mbar to 200 mbar.

6. The process according to at least one of claims 1 to 4, wherein the column top pressure is atmospheric pressure.

7. The process according to at least one of claims 1 to 6, wherein the mixture used in the process also comprises further amines which have a higher boiling point than ethylenediamine, where the weight ratio of ethylenediamine to the higher-boiling amines is in the range from 1:0.1 to 1:1.

8. The process according to at least one of claims 1 to 7, wherein the mixture used in the process has been obtained from the conversion of ethylenediamine from ethanolamine and ammonia.

9. The process according to at least one of claims 1 to 8, wherein the mixture used in the process has been obtained from the conversion of formaldehyde, hydrogen cyanide, ammonia and hydrogen.

10. A process for preparing a distillable mixture comprising ethylenediamine and N-methylethylenediamine, which comprises adding a sufficient amount of water to a mixture comprising ethylenediamine and N-methylethylenediamine that, after the addition of water, the weight ratio of ethylenediamine to water is a*X : Y where X is the proportion by weight of water and Y is the proportion by weight of ethylenediamine at the azeotropic point of a binary mixture of water and ethylenediamine at the column top pressure at which a subsequent distillation of the distillable mixture according to any of claims 1 to 9 can be effected, and a is a real number with a value of 0.9 to 2.0.

11. The process according to claim 10, wherein the distillable mixture is distilled in a distillation column at a pressure of 10 mbar to 4 bar.

## Revendications

1. Procédé de distillation d'un mélange contenant de l'eau, de l'éthylènediamine et de la N-méthyléthylènediamine, le mélange étant introduit dans une colonne de distillation qui est exploitée à une pression en tête de colonne de 10 mbars à 4 bars, **caractérisé en ce que** le rapport pondéral de l'eau à l'éthylènediamine dans le mélange utilisé vaut a * X:Y, où X représente la proportion pondérale d'eau et Y la proportion pondérale d'éthylènediamine au point azéotropique d'un mélange binaire d'eau et d'éthylènediamine à la pression en tête de colonne concernée et a représente un nombre réel d'une valeur de 0,9 à 2,0.

2. Procédé selon la revendication 1, **caractérisé en ce que** a représente un nombre réel d'une valeur de 1,01 à 1, 2.

3. Procédé selon au moins l'une quelconque des revendications 1 à 2, **caractérisé en ce que** le mélange utilisé dans le procédé contient moins de 5% en poids d'ammoniaque.

4. Procédé selon au moins l'une quelconque des revendications 1 à 3, **caractérisé en ce que** le rapport pondéral d'éthylènediamine à N-méthyléthylènediamine dans le mélange utilisé se situe dans la plage de 1:0,005 à 1:0,1.

5. Procédé selon au moins l'une quelconque des revendications 1 à 4, **caractérisé en ce que** la pression en tête de colonne est de 50 mbars à 200 mbars.

6. Procédé selon au moins l'une quelconque des revendications 1 à 4, **caractérisé en ce que** la pression en tête de colonne est la pression atmosphérique.

7. Procédé selon au moins l'une quelconque des revendications 1 à 6, **caractérisé en ce que** le mélange utilisé dans le procédé contient encore d'autres amines, qui présentent un point d'ébullition supérieur à celui de l'éthylènediamine, le rapport pondéral d'éthylènediamine aux amines à point d'ébullition supérieur se situant dans la plage de 1:0,1 à 1:1.

8. Procédé selon au moins l'une quelconque des revendications 1 à 7, **caractérisé en ce que** le mélange utilisé dans le procédé est obtenu de la transformation d'éthylènediamine à partir d'éthanolamine et d'ammoniaque.

9. Procédé selon au moins l'une quelconque des revendications 1 à 8, **caractérisé en ce que** le mélange utilisé dans le procédé est obtenu à partir de la transformation de formaldéhyde, d'acide cyanhydrique, d'ammoniaque et d'hydrogène.

10. Procédé pour la préparation d'un mélange distillable, contenant de l'éthylènediamine et de la N-méthyléthylènediamine, **caractérisé en ce qu'**on ajoute, à un mélange contenant de l'éthylènediamine et de la N-méthyléthylènediamine, une quantité d'eau telle qu'après l'addition d'eau, le rapport pondéral d'éthylènediamine à eau vaut a * X:Y, où X représente la proportion pondérale d'eau et Y la proportion pondérale d'éthylènediamine au point azéotropique d'un mélange binaire d'eau et d'éthylènediamine à la pression en tête de colonne, à laquelle une distillation consécutive du mélange distillable peut avoir lieu selon l'une quelconque des revendications 1 à 9, et a représente un nombre réel d'un valeur de 0,9 à 2,0.

11. Procédé selon la revendication 10, **caractérisé en ce que** le mélange distillable est distillé dans la colonne de distillation à une pression de 10 mbars à 4 bars.
